# FASCICULE DE BREVET EUROPEEN

(11) **EP 0 422 984 B1**
(45) Date de publication et mention de la délivrance du brevet: **29.09.1993**
(21) Numéro de dépôt: 90402714.1
(22) Date de dépôt: 01.10.1990
(51) Int. Cl.: A61K 7/48, A61K 31/08

(54) **Système ternaire à base d'éthers perfluorés**
Ternäres System auf der Basis von Perfluoräther
Ternary system based on perfluorethers

(30) Priorité: 13.10.1989 FR 8913426
(43) Date de publication de la demande: 17.04.1991
(73) Titulaire: YVES SAINT LAURENT PARFUMS, (Société Anonyme), 92200 Neuilly sur Seine (FR)
(72) Inventeur: Seu Salerno,Martine, Yves Saint Laurent Parfums, F-78170 La Celle Saint Cloud (FR); Mounier, Rémy, Yves Saint Laurent Parfums, F-78170 La Celle Saint Cloud (FR); Breda, Bernard, Yves Saint Laurent Parfums, F-78170 La Celle Saint Cloud (FR)
(74) Mandataire: Lerner, François

(56) Documents cités:
- EP-A- 0 196 904
- DE-A- 2 319 971
- GB-A- 2 065 687
- GB-A- 2 206 048
- CHEMICAL ABSTRACTS, vol. 111, no. 16, 16 octobre 1989 Columbus, Ohio, USA Kazuhiko Shimizu et al.: "Perfluoropolyether-containing cosmetics with good moisturizing properties" page 401; colonne 2; ref. no. 14046, & JP-A-63 107 911

## Description

L'invention a pour objet un nouveau système ternaire constitué autour d'un liquide perfluoré et destiné en particulier à une application dans le domaine de la cosmétique esthétique et de soin, afin de protéger la peau en la laissant respirer.

Bien entendu, il a déjà été proposé de nombreux systèmes et de nombreuses compositions cosmétiques pour le traitement ou l'embellissement de la peau ; mais jusqu'à présent ceux-ci se sont avérés dans un certain nombre de cas peu efficaces sur le plan des actions combinées d'hydratation et de protection de la peau, en association avec la nécessaire respiration de cette dernière.

Or, associer ces trois principes essentielles que sont l'hydratation, la protection et la respiration de la peau au sein d'un même système, apparaît délicat à réaliser, compte-tenu des caractéristiques chimiques parfois difficilement compatibles propres à chaque constituant du système.

La composition, de type émulsion, divulguée au document EP-A-0 196 904 permet apparemment d'obtenir des résultats quant à la respiration, à la protection et à l'hydratation de la peau.

En fait, cette composition est telle qu'elle comprend trois phases non miscibles entre-elles, constituées par :
- une première phase d'éthers perfluorés,
- une seconde phase aqueuse,
- et une troisième phase lipophile.

Et cette phase lipophile est présentée comme étant constituée d'huile "classique", à base de glycérides ou d'hydrocarbures.

Pour intéressante qu'elle soit, une composition avec une telle phase lipophile ne semble pas permettre d'optimiser l'action sur la peau des éthers perfluorés en valorisant les qualités d'ensemble de la composition.

L'invention propose une solution à cela ; à savoir, réaliser ladite phase lipophile à base de silicones pour qu'elle soit constituée des silicones comprenant au moins une silicone à chaine linéaire et à groupement glycol, utilisée en tant qu'agent tensio-actif, et au moins une silicone à chaîne cyclique et à groupement méthyle utilisée en tant que base fluide.

De cette façon, les utilisatrices, soucieuses notamment de prodiguer à leur visage des soins de peau réguliers, auront à leur disposition un produit cosmétique à texture non grasse, assurant une protection non-occlusive de leur peau et dont la quantité d'éthers perfluorés déposée sur cette dernière pourra être optimisée par volatilisation très rapide de la partie hydro-siliconée du système.

Bien entendu, il existe déjà des compositions cosmétiques utilisant des silicones. Celle du document GB-A-2 206 048 en est un exemple. Toutefois, il s'agit là d'obtenir une composition multicouches qui demeure en émulsion homogène pendant au moins 30 secondes. Tel n'est pas le but que s'est fixée l'invention. De surcroît, la composition en cause ne comporte que deux phases (eau/silicone). Elle ne donne donc notamment aucun renseignement quant à la compatibilité des silicones utilisées et en particulier des éthers perfluorés, cette compatibilité n'étant aucunement évidente d'emblée compte-tenu de ce que l'on sait de ces produits.

Or l'invention prouve justement que l'on peut obtenir une émulsion stable, valorisante, en associant des éthers perfluorés et des silicones.

Selon l'invention, l'éther perfluoré utilisé sera d'ailleurs de préférence un éther de perfluoropolyméthylisopropyl, dont la formule chimique est la suivante : 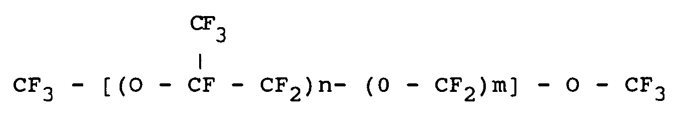
avec n/m = 20 - 40

Cet éther est également connu sous le nom chimique simplifié de perfluoropolyéther et est fabriqué notamment par la Société "MONTEFLUOS" sous l'appelation "FOMBLIN HC" (marque déposée).

Avantageusement, l'éther perfluoré choisi aura une masse moléculaire moyenne oscillant entre 1 500 et 6 600, avec une tension de vapeur de préférence inférieure ou égale à mm Hg environ (soit environ 131 X 10⁻⁷ Pa, pour une masse moléculaire de l'ordre de 6 600) et de toutes façons inférieure à 10⁻³ mm Hg, soit de l'ordre de 131 X 10⁻³ Pa (pour une masse moléculaire de l'ordre de 1 500). Quant à la tension de surface prise à 20°C, elle sera de l'ordre de 20 à 25 mN/m.

Parmi les silicones prévues dans l'invention pour être associées à l'éther perfluoré retenu, on trouvera donc nécessairement au moins une silicone à chaîne(s) linéaire(s)) prévue en tant qu'agent tensio-actif et au moins une silicone (de préférence volatile) à chaîne cyclique destinée à constituer une base fluide.

Dans la première catégorie (silicones linéaires), les silicones de type copolyol de dimethicone sont tout particulièrement appropriées de même que les cyclométhicones (designation CTFA)dans la seconde catégorie (silicone cyclique).

Les copolyols de dimethicone sont des copolymères de glycol solubles dans l'eau, dans l'alcool et dans les sytèmes hydro-alcooliques. En d'autres termes, il s'agit de polymères de diméthylsiloxane comprenant des chaînes latérales de polyoxyéthylène et/ou de polyoxypropylène.

Ce type de silicone est commercialisée notamment par la Société "DOW CORNING".

Quant aux cyclométhicones, il s'agit de composés de diméthyl volatils polysiloxanes qui se présentent sous l'aspect physique d'un fluide à faible viscosité (de l'ordre de 2,5 à 6 mm²/s. à 25°C) avec une tension de surface de l'ordre de 18 à 21 mN/m (également à 25°C environ).

La formule chimique des cyclométhicones est la suivante : 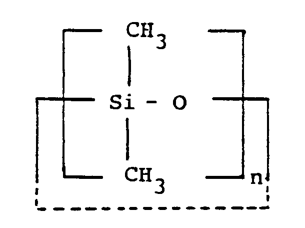
avec n (entier naturel) compris entre 3 et 6

Dans l'invention, on utilisera ce produit de préférence sous sa forme tétramère et/ou pentamère, essentiellement. On pourrait toutefois également envisager de l'inclure en moindre quantité sous sa forme hexamère.

Les cyclométhicones ont notamment été retenues car elles présentent l'avantage d'agir efficacement en tant qu'agent de nettoyage de la peau, sans procurer de sensation d'échauffement.

En tant que base fluide ou agent porteur transitoire elles permettent d'obtenir un produit facile à étendre et à retirer et dont la diffusion est aisée.

Un tel produit est notamment commercialisé par la Société "DOW CORNING" sous la dénomination "DC 344" ou "DC 345".

Selon l'invention, les différents ingrédients qui viennent d'être cités se retrouvent de préférence dans le système ternaire final dans les concentrations suivantes :
. éthers perfluorés : entre 0,1 et 5 % en poids environ,
. silicones à chaîne(s) linéaire(s) (copolyol de diméthicone) : entre 0,1 et 5 % environ,
. silicones cycliques (cyclométhicone) : entre 0,1 et 50 % en poids environ.

Il est à noter qu'éventuellement les copolyols de diméthicone et les cyclomethicones pourront être, pour partie, regroupées au sein d'un produit unique du type silicone volatile auto-émulsionnable.

Une telle silicone est par exemple commercialisée par la Société "DOW CORNING" sous la dénomination "DC 3225" .

Elle se présente sous l'aspect d'un liquide qui varie entre la transparence et un aspect légèrement opaque ou troublé.

Dans ce produit, on retrouve environ 10 % de copolyol de dimethicone et environ 90 % de cyclomethicone.

On notera par ailleurs également qu'avantageusement la phase aqueuse du système ternaire de l'invention comprendra en tant qu'additifs, ou adjuvants, un agent hydratant, un co-émulsionnant (ou émulsionnant d'adjonction) constitué par un électrolyte ainsi qu'un agent conservateur anti-bactérien.

L'émulsionnant d'adjonction pourra être constitué par du chlorure de sodium, voire éventuellement du chlorure de potassium.

Quant aux agents hydratants et conservateurs anti-bactérien, ils pourront respectivement être constitués à base de glycérine et d'imidazolidinyl urée, ce dernier constituant étant fabriqué notamment par la société "ADF chimie" sous l'appellation"GERMALL 115."

On va maintenant donner à titre d'illustration non limitative deux exemples de réalisation d'un système ternaire conforme à l'invention :

### Exemple 1.

Pour obtenir un système présentant les caracréristiques recherchées de l'invention on procède au mélange des ingredients suivants qui sont ainsi dosés en % poids/poids :
- Copolyol de dimethicone : entre 1 et 1,5 % environ,
- cyclométhicone de type tétramère : entre 14 et 23,5 % environ,
- cyclométhicone de type pentamère : entre 5 et 10 %, environ,
- Ether de perfluoropolyméthylisopropyl : entre 0,5 et 1 % environ,
- Co-émulsionnant (chlorure de sodium) de l'ordre de 2 %,
- Agent hydratant (glycérine) entre 3 et 5 % environ,
- Agent conservateur anti-bactérien : de l'ordre de 0,3 %,
- Eau, Q.S.P. 100.

### Exemple 2.

On mélange maintenant les ingredients de la façon suivante, en % poids/poids :
- association de copolyol de diméthicone (environ 10 %) et de cyclométhicone de type tétramère (environ 90 %) (DC 3225) : 10 % environ,
- cyclométhicone de type tétramère (DC 344) : 5 % environ,
- cyclométhicone de type pentamère (DC 345) : 5 % environ,
- Ether perfluoré (FOMBLIN HC) : 0,5 % environ,
- Agent conservateur antibactérien : 0,3 % environ,
- Glycérine : 3 %
- Chlorure de Sodium : 2 % environ,
- Eau distillé : 74,2 %.

## Revendications

1. Composition cosmétique de type émulsion comprenant trois phases non miscibles entre-elles, constituées par :
- une première phase d'éthers perfluorés,
- une seconde phase aqueuse,
- et une troisième phase de silicones comprenant au moins une silicone à chaine linéaire et à groupement glycol, utilisée en tant qu'agent tensio-actif, et au moins une silicone à chaîne cyclique et à groupement méthyle utilisée en tant que base fluide.

2. Composition selon la revendication 1 caractérisée en ce que la silicone à chaîne linéaire et à groupement glycol est du type copolyol de diméthicone.

3. Composition selon la revendication 1 ou la revendication 2 caractérisée en ce que la silicone à chaîne cyclique et à groupement méthyle est du type cyclométhicone.

4. Composition selon la revendication 3 caractérisée en ce que la silicone de type cyclométhicone est présente dans le système sous sa forme tétramère et pentamère essentiellement.

5. Composition selon l'une quelconque des revendications précédentes caractérisée en ce que la phase d'éthers perfluorés comprend un éther de perfluoropolyméthylisopropyl.

6. Composition selon l'une quelconque des revendications précédentes caractérisée en ce que :
- la phase d'éthers perfluorés constitue entre 0,1 et 5 % en poids environ de la composition, et
- la phase de silicones comprend lesdites silicones à chaîne linéaire et à groupement glycol, d'une part, et à chaîne cyclique et à groupement méthyle d'autre part, ces silicones représentant respectivement entre 0,1 et 5 % en poids environ et entre 0,1 et 50 % en poids environ de la composition.

7. composition selon l'une quelconque des revendications précédentes caractérisée en ce que la phase aqueuse contient :
- un agent hydratant,
- un co-émulsionnant constitué par un électrolyte,
- et un agent conservateur anti-bactérien.

8. Composition selon la revendication 7 caractérisée en ce que le co-émulsionnant comprend du chlorure de sodium.

9. Composition selon la revendication 7 ou la revendication 8 caractérisée en ce que l'agent hydratant comprend de la glycérine et en ce que l'agent conservateur anti-bactérien comprend de l'imidazolidinyl urée.

10. Composition selon l'une quelconque des revendications 7 à 9, caractérisée en ce qu'elle comprend, en % poids/poids :
- entre 1 et 1,5 % environ de copolyol de diméthicone,
- entre 14 et 23,5 % environ de cyclométhicone de type tétramère,
- entre 5 et 10 % environ de cyclomethicone de type pentamère,
- entre 0,5 et 1 % environ d'éther de perfluoropolyméthylisopropyl,
- environ 2 % de co-émulsionnant,
- entre 3 et 5 % environ d'agent hydratant,
- de l'ordre de 0,3 % d'agent conservateur antibactérien.
- de l'eau, Q.S.P. 100.

## Patentansprüche

1. Kosmetische Zusammensetzung vom Emulsionstyp mit drei miteinander nicht mischbaren Phasen, bestehend aus:
- einer ersten Phase von perfluorierten Ethern,
- einer zweiten wässrigen Phase
- und einer dritten Phase von Silikonen, die wenigstens ein Silikon mit gerader Kette und Glycolgruppe, das als oberflächenaktives Mittel benutzt wird, und wenigstens ein Silikon mit zyklischer Kette und Methylgruppe, das als Fließmittelbasis benutzt wird, umfaßt.

2. Zusammensetzung nach Anspruch 1, **dadurch gekennzeichnet**, daß das Silikon mit gerader Kette und Glycolgruppe vom Dimethiconcopolyoltyp ist.

3. Zusammensetzung nach Anspruch 1 oder Anspruch 2, **dadurch gekennzeichnet,** daß das Silikon mit zyklischer Kette und Methylgruppe vom Cyclomethicontyp ist.

4. Zusammensetzung nach Anspruch 3, **dadurch gekennzeichnet,** daß das Silikon vom Cyclomethicontyp in dem System im wesentlichen in seiner Tetramer- und Pentamerform vorliegt.

5. Zusammensetzung nach einem der vorausgehenden Ansprüche, **dadurch** **gekenn****zeichnet,** daß die Phase der perfluorierten Ether einen Perfluorpolymethylisopropylether umfaßt.

6. Zusammensetzung nach einem der vorausgehenden Ansprüche, **dadurch** **gekenn****zeichnet,** daß:
- die Phase der perfluorierten Ether etwa 0,1 bis 5 Gew.-% der Zusammensetzung ausmacht und
- die Phase der Silikone die Silikone mit gerader Kette und Glycolgruppe einerseits und mit zyklischer Kette und Methylgruppe andererseits umfaßt, die jeweils etwa 0,1 bis 5 Gew.-% bzw. etwa 0,1 bis 50 Gew.-% der Zusammensetzung ausmachen.

7. Zusammensetzung nach einem der vorausgehenden Ansprüche, **dadurch gekenn****zeichnet**, daß die wässrige Phase:
- ein hydratisierendes Mittel,
- einen Coemulgator, der von einem Elektrolyten gebildet wird,
- und ein antibakterielles Konservierungsmittel enthält.

8. Zusammensetzung nach Anspruch 7, **dadurch** **gekennzeichnet,** daß der Coemulgator Natriumchlorid umfaßt.

9. Zusammensetzung nach Anspruch 7 oder Anspruch 8, **dadurch gekennzeichnet**, daß das hydratisierende Mittel Glyzerin umfaßt und daß das antibakterielle Konservierungsmittel Imidazolidinylharnstoff umfaßt.

10. Zusammensetzung nach einem der Ansprüche 7 bis 9, **dadurch gekennzeichnet**, daß sie in Gewichts-/Gewichts-Prozenten:
- etwa 1 bis 1,5% Dimethiconcopolyol,
- etwa 14 bis 23,5% Cyclomethicon vom Tetramertyp,
- etwa 5 bis 10% Cyclomethicon vom Pentamertyp,
- etwa 0,5 bis 1% Perfluorpolymethylisopropylether,
- etwa 2% Coemulgator,
- etwa 3 bis 5% hydratisierendes Mittel,
- antibakterielles Konservierungsmittel in der Größenordnung von 0,3% und
- Wasser Q.S.P. 100 umfaßt.

## Claims

1. Cosmetic composition of the emulsion type, comprising three phases which are immiscible with one another, consisting of:
- a first phase of perfluorinated ethers;
- a second, aqueous phase; and
- a third phase of silicones comprising at least one linear chain silicone with a glycol group, used as a surfactant, and at least one cyclical chain silicone with a methyl group used as a fluid base.

2. Composition according to Claim 1, characterised in that the linear chain silicone with the glycol group is of the dimethicone copolyol type.

3. Composition according to Claim 1 or Claim 2, characterised in that the cyclical chain silicone with the methyl group is of the cyclomethicone type.

4. Composition according to Claim 3, characterised in that the silicone of the cyclomethicone type is present in the system substantially in its tetrameric and pentameric form.

5. Composition according to any one of the preceding Claims, characterised in that the perfluorinated ether phase comprises a perfluoropolymethylisopropyl ether.

6. Composition according to any one of the preceding Claims, characterised in that:
- the perfluorinated ether phase consists of between approximately 0.1 and 5 weight % of the composition; and
- the silicone phase comprising the said linear chain silicones with glycol groups on the one hand and the cyclical chain silicones with methyl groups on the other hand consist respectively of between approximately 0.1 and 5 weight % and between approximately 0.1 and 50 weight % of the composition.

7. Composition according to any one of the preceding Claims, characterised in that the aqueous phase contains:
- a moisturiser;
- a co-emulsifier consisting of an electrolyte; and
- an anti-bacterial preservative.

8. Composition according to Claim 7, characterised in that the co-emulsifier comprises sodium chloride.

9. Composition according to Claim 7 or Claim 8, characterised in that the moisturiser comprises glycerol and the anti-bacterial preservative comprises imidazolidinyl urea.

10. Composition according to any one of Claims 7 to 9, characterised in that it comprises the following in weight %/ %:
- between approximately 1 and 1.5% dimethicone copolyol;
- between approximately 14 and 23.5% cyclomethicone of the tetrameric type;
- between approximately 5 and 10% cyclomethicone of the pentameric type;
- between approximately 0.5 and 1% perfluoropolymethylisopropyl ether;
- approximately 2% co-emulsifier;
- between approximately 3 and 5% moisturiser;
- approximately 0.3% anti-bacterial preservative; and
- water Q.S.P. 100.
